# EUROPÄISCHE PATENTANMELDUNG

(11) **EP 4 796 044 A1**
(43) Veröffentlichungstag der Anmeldung: **26.08.2026**
(21) Anmeldenummer: 25159872.8
(22) Anmeldetag: 25.02.2025
(51) Int. Cl.: A61B 6/04, A61N 5/10

(54) **VORRICHTUNG ZUR LAGERUNG EINES PATIENTEN MIT SCHWENKBAR BEFESTIGTEM HARDWAREMODUL**

(71) Anmelder: Siemens Healthineers AG, 91301 Forchheim (DE)
(72) Erfinder: HRUSCHKA, Klaus, 95478 Kemnath (DE); BAUMANN, Berthold, 95478 Kemnath (DE); EIGNER, Daniel, 95478 Kemnath (DE); STOCK, Johannes, 95478 Kemnath (DE); BUCHENAUER, Andreas, 95478 Kemnath (DE); BARTHEL, Markus, 95478 Kemnath (DE); KNODT, Konstantin, 95478 Kemnath (DE); NEUBER, Wolfgang, 95478 Kemnath (DE); SCHMÄLZLE, Thomas, 95478 Kemnath (DE); PLANNERER, Patrick, 95478 Kemnath (DE); BUCHBINDER, Stefan, 95478 Kemnath (DE); KILIAN, Katrin, 95478 Kemnath (DE); HERZOG, Urs, 5405 Dättwil (CH)
(74) Vertreter: Siemens Healthineers Patent Attorneys

(57) **Zusammenfassung**

Die Erfindung betrifft eine Vorrichtung (1) zur Lagerung eines Patienten. Dabei weist die Vorrichtung (1) eine Säule (5), eine Patientenauflage (4) und ein Hardwaremodul (6) auf. Das Hardwaremodul (6) weist eine Steuerungselektronik (8) zur Ansteuerung wenigstens eines Aktorelements zur räumlichen Lageveränderung der Patientenauflage (4) auf. Das Hardwaremodul (6) weist außerdem einen Träger (9) auf, auf dem die Steuerungselektronik (8) angeordnet ist. Das Hardwaremodul (6) ist dabei um eine Rotationsachse (7) schwenkbar an der Säule (5) befestigt. Das Hardwaremodul (6) ist in eine erste Schwenkstellung positionierbar, in der sich die Steuerungselektronik (8) in einem Innenraum der Säule (5) befindet und in eine zweite Schwenkstellung positionierbar, in der die Steuerungselektronik (8) von außerhalb der Säule (5) zugänglich ist.

## Beschreibung

Die vorliegende Erfindung betrifft eine Vorrichtung zur Lagerung eines Patienten, beispielsweise zur Lagerung während einer Strahlentherapie mittels eines Bestrahlungssystems oder während einer Erzeugung von Bilddaten mittels eines Bildgebungssystems. Die Erfindung betrifft ferner ein entsprechendes Bestrahlungssystem und ein Bildgebungssystem.

Vorrichtungen zur Lagerung eines Patienten, insbesondere für die Strahlentherapie, Radiologie oder Fluoroskopie, und insbesondere mit verstellbaren Patientenauflagen sind aus dem Stand der Technik bekannt und werden unter anderem als Patientenliegen, Patiententische oder Patientenlagerungen bezeichnet. Die Vorrichtung kann beispielweise dazu dienen, einen untersuchungsrelevanten Körperbereich eines Patienten insbesondere in einen vorgegebenen Zielbereich, beispielweise in einen Strahlengang einer Strahlungsquelle zur Bestrahlung des Patienten oder zwischen einen Röntgenstrahler und einen Röntgendetektor für die Bilderfassung oder in eine Röhre eines MRT-Geräts, geeignet zur positionieren.

Für die Lagerung des Patienten kann es vorteilhaft sein, eine Lage des untersuchungsrelevanten Körperbereichs variieren zu können, beispielsweise bezüglich möglichst vieler Raumrichtungen und/oder Rotationsachsen variieren zu können. Zur Variation der Lage sind Aktorelemente bekannt, die im Umfeld der Patientenauflage montiert sind und beispielsweise mit einer Steuerungselektronik verbunden sind. Wird eine Anzahl der zu variierenden Raumrichtungen und/oder zu variierenden Rotationsachsen erhöht, erhöht sich konsequent ein Bauraumbedarf der Aktorelemente und/oder der Steuerungselektronik. Ein vorhandener Bauraum innerhalb der Vorrichtung, also beispielsweise im Bereich unterhalb der Patientenauflage, ist insbesondere begrenzt und/oder steht nicht für den Einbau der erhöhten Anzahl an Aktorelementen und/oder der Steuerungselektronik zur Verfügung.

Dieser Herausforderung wird im Stand der Technik damit begegnet, dass Teile der Aktorelemente und/oder der Steuerungselektronik dezentral, also beispielsweise außerhalb der Vorrichtung verbaut werden. Alternativ wird auch der vorhandene Bauraum durch eine Vergrößerung der Vorrichtung erweitert, was allerdings nicht für alle Anwendungsfälle möglich ist. Außerdem kann in bekannten Lösungen die Zugänglichkeit zum Beispiel der Steuerungselektronik eingeschränkt sein.

Es ist daher eine Aufgabe der vorliegenden Erfindung, ein verbessertes Konzept zur Integration einer Steuerungselektronik in einer Vorrichtung zur Lagerung eines Patienten anzugeben, durch das ein vorhandener Bauraum verbessert genutzt wird und durch das eine einfache Zugänglichkeit der Steuerungselektronik gewährleistet ist.

Diese Aufgabe wird gelöst durch den unabhängigen Anspruch. Vorteilhafte Weiterbildungen und bevorzugte Ausführungsformen sind Gegenstand der abhängigen Ansprüche.

Das verbesserte Konzept beruht auf der Idee, die Steuerungselektronik über eine schwenkbare Lösung innerhalb der Vorrichtung zu integrieren.

Gemäß einem Aspekt der Erfindung wird eine Vorrichtung zur Lagerung eines Patienten vorgestellt. Die Vorrichtung weist eine Säule, eine, insbesondere auf der Säule montierte, Patientenauflage, und ein Hardwaremodul auf. Das Hardwaremodul weist eine Steuerungselektronik zur Ansteuerung wenigstens eines Aktorelements zur räumlichen Lageveränderung der Patientenauflage auf. Das Hardwaremodul weist außerdem einen Träger auf, auf dem die Steuerungselektronik angeordnet ist. Das Hardwaremodul, also zumindest der Träger und die Steuerungselektronik, ist dabei um eine Rotationsachse schwenkbar an der Säule befestigt und das Hardwaremodul ist in eine erste Schwenkstellung positionierbar, in der sich die Steuerungselektronik in einem Innenraum der Säule befindet. Außerdem ist das Hardwaremodul in eine zweite Schwenkstellung positionierbar, in der die Steuerungselektronik von außerhalb der Säule zugänglich ist.

Der Patient kann dabei insbesondere eine Person sein, an der eine Untersuchung und/oder eine Behandlung durchgeführt werden soll. Es soll insbesondere möglich sein, den Patienten oder zumindest einen Teil des Patienten in eine für die jeweilige Untersuchung und/oder Behandlung vorteilhafte Lage zu bringen. Dazu ist zum Beispiel eine Lageveränderung der Patientenauflage notwendig.

Die Patientenauflage kann insbesondere derart ausgebildet sein, dass sich der Patient auf die Patientenauflage legen, setzen oder stellen kann. Die Patientenauflage kann eine Liegefläche, eine Sitzfläche und/oder eine Fläche zum Anlehnen aufweisen, die zur Positionierung des Patienten dienen kann. Die Lageveränderung der Patientenauflage kann dabei durch eine Veränderung der Position und/oder eine Veränderung der Orientierung der Patientenauflage stattfinden. Die Patientenauflage kann insbesondere beweglich an oder auf der Säule befestigt sein. Bei bestimmungsgemäßer Aufstellung kann sich die Säule dabei beispielsweise unterhalb oder teilweise unterhalb der Patientenauflage befinden.

Die Säule kann ein beispielsweise ortsfester Bestandteil der Vorrichtung sein und beispielsweise auch als Standfuß, Pfeiler oder Unterbau der Patientenauflage bezeichnet werden. Insbesondere kann die Säule einen weiteren Innenraum aufweisen, der beispielsweise elektrische Leitungen, das wenigstens eine Aktorelement oder weitere Bauteile aufnehmen kann. Beispielsweise kann die Säule wenigstens ein Stützelement aufweisen oder aus dem wenigstens einen Stützelement bestehen, um die Patientenauflage stabil in einer vorbestimmten Lage zu halten. Insbesondere kann die Säule bei bestimmungsgemäßer Aufstellung mit einem Aufstellbereich in Kontakt stehen oder fest mit dem Aufstellbereich verbunden sein.

Insbesondere kann die Vorrichtung zusätzlich das wenigstens eine Aktorelement aufweisen. Das wenigstens eine Aktorelement kann beispielsweise in einem Innenraum der Patientenauflage oder in dem Innenraum der Säule angeordnet sein. Ebenso kann sich ein erstes Aktorelement des wenigstens einen Aktorelements in dem Innenraum der Patientenauflage und ein zweites Aktorelement des wenigstens einen Aktorelements in dem Innenraum der Säule befinden.

Das wenigstens eine Aktorelement kann ebenso als wenigstens ein Aktor oder wenigstens ein Aktuator bezeichnet werden. Das wenigstens eine Aktorelement kann als antriebstechnische Baueinheit bezeichnet werden und dazu eingerichtet sein, ein Signal in eine mechanische Bewegung, Kraft, Druck oder Drehmoment umzusetzen. Beispielsweise kann das wenigstens eine Aktorelement einen Elektromotor, einen hydraulischen Aktor, einen pneumatischen Aktor, einen Stellmotor und/oder einen Zylinder enthalten. Insbesondere kann das wenigstens eine Aktorelement unterschiedlich Arten von Aktoren enthalten, um beispielsweise die Veränderung der Position und/oder die Veränderung der Orientierung hervorzurufen.

Die Steuerungselektronik kann insbesondere wenigstens eine elektronische Schaltung, insbesondere eine Leiterplatte mit montierten elektronischen Bauteilen, und optional ein Gehäuse enthalten. Der Träger kann dabei beispielsweise einer tragenden Gehäusestruktur entsprechen, die beispielsweise zu einer Seite geöffnet ist oder wenigstens eine weitere Öffnung aufweist. Ebenso kann der Träger wenigstens einen Anschlussstecker, Kabel und weitere Montagemöglichkeiten aufweisen. Alternativ kann der Träger auch eine Leiterplatte aufweisen oder aus einem Leiterplattenmaterial bestehen.

Insbesondere ist der Träger ausschließlich an der Rotationsachse an der Säule befestigt. Alternativ kann der Träger zur Befestigung an der Säule zusätzlich zur Befestigung an der Rotationsachse eine Führungsschiene oder eine Gleitschiene enthalten, die sich zum Beispiel an einer Seite des Trägers befindet, die der Rotationsachse gegenüberliegt.

Bei bestimmungsgemäßem Aufbau kann sich der Innenraum der Säule beispielsweise unterhalb der Patientenauflage befinden. Insbesondere kann die Säule beispielsweise ein Säulengehäuse aufweisen, das den Zugang zum Innenraum verhindert, wenn sich das Hardwaremodul in der ersten Schwenkstellung befindet. Mit anderen Worten kann der Zugang zum Beispiel zu dem Hardwaremodul und/oder zu der Steuerungselektronik nur eingeschränkt oder gar nicht möglich sein, wenn sich das Hardwaremodul in der ersten Schwenkstellung befindet.

Der Zugang zu dem Hardwaremodul und/oder zu der Steuerungselektronik kann es insbesondere ermöglichen, die Steuerungselektronik beispielsweise mit einem Werkzeug oder mit einer Hand zu erreichen, um beispielsweise ein Datenkabel zum Auslesen und/oder Aufspielen von Daten anzuschließen. Ebenso kann der Zugang bedeuten, dass Bestandteile der Steuerungselektronik, wie elektronische Bauteile oder Steckmodule geprüft, getauscht oder repariert werden können. Dazu kann es notwendig oder vorteilhaft sein, dass sich wenigstens ein Teil des Hardwaremoduls außerhalb der Säule befindet. Ein weiterer Teil, der sich beispielsweise in der zweiten Schwenkstellung in dem Innenraum der Säule befindet, kann insbesondere einen eingeschränkten Zugang haben, da zum Beispiel eine Führung notwendig ist, um die oben genannten Schritte durchzuführen. Mit anderen Worten kann sich das Hardwaremodul in der zweiten Schwenkstellung wenigstens zum Teil außerhalb der Säule befinden, sodass die Steuerungselektronik dadurch von außerhalb der Säule zugänglich ist.

Beispielsweise kann es zu Servicezwecken und/oder Testzwecken und/oder Reparaturzwecken notwendig sein, Zugang zur Steuerungselektronik zu erhalten. Insbesondere zu diesen Zwecken kann das Hardwaremodul von der ersten in die zweite Schwenkstellung positioniert werden, insbesondere durch Schwenken um die Rotationsachse, und damit die Steuerungselektronik beispielsweise zum Überprüfen auf Funktionalität oder zum Auslesen von Daten zu erreichen.

Ein Vorteil der Erfindung ist, dass sich das Hardwaremodul in der ersten Schwenkstellung in den Innenraum der Säule schwenken lässt und damit der vorhandene Bauraum genutzt wird, ohne beispielsweise eine äußere Abmessung der Vorrichtung zu vergrößern. Der Zugang zu der Steuerungselektronik ist über die zweite Schwenkstellung gewährleistet und in der zweiten Schwenkstellung sind damit Servicearbeiten und/oder Reparaturarbeiten sowie Tests möglich. Die Vorrichtung kann somit kompakt und platzsparend in Bezug auf den Aufstellbereich ausgelegt werden, ohne dafür einen Kompromiss bei der Zugänglichkeit einzugehen. Ebenso kann eine räumliche Nähe zwischen der Steuerungselektronik und dem wenigstens einen Aktorelement sichergestellt werden.

Ein weiterer Vorteil der Erfindung ist, insbesondere im Vergleich zu Lösungen, die eine translatorische Verschiebbarkeit des Hardwaremoduls im Sinne eines Schubes oder einer Schublade oder dergleichen vorsehen, dass Anschlussleitungen, welche die Steuerungselektronik mit entfernt von dem Hardwaremodul angeordneten Komponenten verbinden, aufgrund der Rotationsbewegung weniger umfänglich bewegt werden müssen. Dies kann unerwünschte Bewegungen oder Belastungen der Anschlussleitungen oder entsprechender Verbindungen der Anschlussleitungen mit der Steuerungselektronik oder dem Hardwaremodul vermeiden oder verringern.

Gemäß zumindest einer Ausführungsform weist das Hardwaremodul ein Anschlagelement auf, das mit dem Träger verbunden ist und das eine Schwenkbewegung des Hardwaremoduls von der ersten Schwenkstellung in die zweite Schwenkstellung in der zweiten Schwenkstellung begrenzt.

Wenn sich das Hardwaremodul in der ersten Schwenkstellung befindet, kann sich das Anschlagelement insbesondere innerhalb des Innenraums der Säule befinden. Während der Schwenkbewegung des Hardwaremoduls kann das Anschlagelement beispielsweise in einer Führungsschiene oder in einer Arretierung verlaufen, beispielsweise mit dem Hardwaremodul mitbewegt werden. In der zweiten Schwenkstellung des Hardwaremoduls kann das Anschlagelement oder zumindest ein Teil des Anschlagelements beispielsweise von außerhalb der Säule zugänglich sein. In der zweiten Schwenkstellung des Hardwaremoduls kann das Anschlagelement beispielsweise durch ein Einrasten oder ein Arretieren an der Säule die Schwenkbewegung des Hardwaremoduls begrenzen.

Ein Vorteil dieser Ausführungsformen ist, dass eine Bewegung des Hardwaremoduls aufgrund des Anschlagelements auf eine definierte Position eingeschränkt werden kann. Das Hardwaremodul kann sich aufgrund der Begrenzung durch das Anschlagelement von der ersten Schwenkposition in die zweite Schwenkposition und zurück in die erste Schwenkposition bewegen. Dadurch kann das Hardwaremodul und damit die Steuerungselektronik zu Reparaturzwecken und/oder Servicezwecken einfach zugänglich gemacht werden.

Gemäß zumindest einer weiteren Ausführungsform ist das Anschlagelement zerstörungsfrei von dem Träger demontierbar und das Hardwaremodul ist nur bei demontiertem Anschlagelement in eine dritte Schwenkposition positionierbar, in der die Steuerungselektronik von dem Träger zerstörungsfrei demontierbar ist. Alternativ ist das Anschlagelement aus einer Arretierung an der Säule lösbar, und das Hardwaremodul ist nur bei aus der Arretierung gelöstem Anschlagelement in eine dritte Schwenkstellung positionierbar, in der die Steuerungselektronik von dem Träger zerstörungsfrei demontierbar ist

Insbesondere ist das Anschlagelement zerstörungsfrei von dem Träger demontierbar und/oder aus der Arretierung lösbar, wenn sich das Hardwaremodul in der zweiten Schwenkstellung befindet. Die dritte Schwenkstellung kann beispielsweise Reparaturzwecken dienen, insbesondere kann in der dritten Schwenkposition die Steuerungselektronik von dem Träger demontiert werden und/oder durch eine weitere Steuerungselektronik ersetzt werden. Ebenso kann in der dritten Schwenkstellung dem Träger beispielsweise eine zusätzliche Steuerungselektronik hinzugefügt werden.

Insbesondere kann das demontierte Anschlagelement in der zweiten Schwenkstellung des Hardwaremoduls montiert werden und/oder die Arretierung an der Säule wiederhergestellt werden.

Ein Vorteil dieser Ausführungsformen ist, dass für eine Reparatur zum Beispiel der Steuerungselektronik eine vereinfachte Zugänglichkeit zu dem Hardwaremodul durch die dritte Schwenkstellung gewährleistet sein kann. Insbesondere kann sich durch die dritte Schwenkstellung eine Servicefähigkeit der Vorrichtung zur Lagerung des Patienten erhöhen.

Gemäß zumindest einer weiteren Ausführungsform ist das Hardwaremodul nur bei demontiertem Anschlagelement von der ersten Schwenkstellung über die zweite Schwenkstellung hinaus in die dritte Schwenkstellung schwenkbar.

Insbesondere kann das Hardwaremodul ebenso von der dritten Schwenkstellung über die zweite Schwenkstellung hinaus in die erste Schwenkstellung schwenkbar sein.

Ein Vorteil dieser Ausführungsformen ist, dass sich das Hardwaremodul jeweils in einer definierten Position befindet. Die erste Schwenkstellung und die zweite Schwenkstellung sind dabei ohne Demontage des Anschlagelements erreichbar. Die dritte Schwenkstellung kann durch die notwendige Demontage des Anschlagelements beispielsweise lediglich für geschultes Servicepersonal erreichbar sein.

Gemäß zumindest einer weiteren Ausführungsform weist die Vorrichtung eine Abdeckung auf, die an der Säule montierbar ist, wenn sich das Hardwaremodul in der ersten Schwenkstellung befindet. Außerdem ist das Hardwaremodul nur bei demontierter Abdeckung von der ersten Schwenkstellung in die zweite Schwenkstellung schwenkbar.

Insbesondere ist das Hardwaremodul von außerhalb des Innenraums der Säule nicht zugänglich, wenn die Abdeckung an der Säule montiert ist. Insbesondere wird durch die Abdeckung das Hardwaremodul abgedeckt und damit beispielsweise vor Umwelteinflüssen wie Staub und/oder Feuchtigkeit geschützt. Außerdem kann das Hardwaremodul vor unbefugtem Zugriff geschützt werden. Insbesondere ist die Abdeckung nicht montierbar, wenn sich das Hardwaremodul in der zweiten Schwenkstellung oder in der dritten Schwenkstellung befindet. Mit anderen Worten wird das Hardwaremodul durch die Montage der Abdeckung an der Säule beispielsweise in die erste Schwenkstellung gezwungen.

Ein Vorteil dieser Ausführungsformen ist, dass durch die Montage der Abdeckung das Hardwaremodul vor Umwelteinflüssen geschützt wird. Ebenso kann das Hardwaremodul durch die montierte Abdeckung vor unbefugtem Zugriff geschützt werden. Zusätzlich kann sich ein ästhetischer Vorteil durch eine Integration der Abdeckung in die Säule ergeben.

Gemäß zumindest einer weiteren Ausführungsform weist die Vorrichtung eine Abdeckung auf, die mit dem Hardwaremodul verbunden ist und die zusammen mit dem Hardwaremodul von der ersten Schwenkstellung in die zweite Schwenkstellung schwenkbar ist.

In der ersten Schwenkstellung kann die Abdeckung das Hardwaremodul abdecken und damit vor Umwelteinflüssen und unbefugtem Zugriff schützen. Insbesondere kann die Abdeckung bei demontiertem Anschlagelement zusammen mit dem Hardwaremodul von der zweiten Schwenkstellung in die dritte Schwenkstellung schwenkbar sein. Die Abdeckung kann insbesondere mit dem Träger verbunden sein, beispielsweise durch eine Verschraubung. Insbesondere kann die Abdeckung alternativ Teil des Trägers sein.

Ein Vorteil dieser Ausführungsformen ist, dass das Hardwaremodul ohne Demontage der Abdeckung von der ersten Schwenkstellung in die zweite Schwenkstellung schwenkbar ist. Dies kann zu einer schnellen und einfachen Servicefähigkeit des Hardwaremoduls führen.

Gemäß zumindest einer weiteren Ausführungsform weist die Vorrichtung eine weitere Abdeckung auf, die mit der Säule verbunden ist und die mit der Abdeckung fluchtet, wenn sich das Hardwaremodul in der ersten Schwenkstellung befindet.

Die Abdeckung und/oder die weitere Abdeckung können im Wesentlichen beispielsweise plane Oberflächen aufweisen. Die weitere Abdeckung und die Abdeckung können beispielsweise bündig ausgeführt sein, wenn sich das Hardwaremodul in der ersten Schwenkstellung befindet. Insbesondere ist dabei die Abdeckung an der Säule montiert.

Ein Vorteil dieser Ausführungsformen ist, dass der Innenraum der Säule vor Umwelteinflüssen und/oder vor unbefugtem Zugriff geschützt werden kann, wenn die Abdeckung und die weitere Abdeckung fluchten. Zusätzlich kann sich durch die beschriebenen Ausführungsformen ein ästhetischer Vorteil für die Vorrichtung ergeben.

Gemäß zumindest einer weiteren Ausführungsform verläuft die Rotationsachse, insbesondere bei bestimmungsgemäßer Aufstellung der Vorrichtung, entlang einer Vertikalrichtung der Vorrichtung.

Insbesondere kann die Schwenkbewegung des Hardwaremoduls von der ersten Schwenkstellung in die zweite Schwenkstellung bei bestimmungsgemäßer Aufstellung der Vorrichtung in einer Horizontalebene senkrecht zu der Vertikalrichtung der Vorrichtung verlaufen. Insbesondere kann ebenso die Schwenkbewegung von der zweiten Schwenkstellung in die dritte Schwenkstellung in der Horizontalebene der Vorrichtung verlaufen.

Ein Vorteil dieser Ausführungsformen ist, dass die Schwenkbewegung des Hardwaremoduls beispielsweise mit einem geringen Kraftaufwand möglich ist, da insbesondere keine Bewegung in vertikaler Richtung notwendig ist. Die Schwenkbewegung ist damit weniger abhängig von einem Gewicht des Hardwaremoduls.

Gemäß zumindest einer weiteren Ausführungsform weist das Hardwaremodul eine elektrische Anschlussleitung zum Anschluss der Steuerungselektronik an das wenigstens eine Aktorelement auf. Außerdem verläuft die elektrische Anschlussleitung von der Rotationsachse entlang einer Oberfläche des Trägers zu der Steuerungselektronik.

Die elektrische Anschlussleitung kann beispielsweise auf einer Seite des Hardwaremoduls verlaufen, die zum Innenraum der Säule zeigt, wenn sich das Hardwaremodul in der ersten Schwenkstellung befindet. Insbesondere kann die elektrische Anschlussleitung darüber hinaus innerhalb des Innenraums der Säule verlaufen. Beispielsweise hat der Träger eine rechteckige Form und die elektrische Anschlussleitung verläuft an einer kurzen Seite des Trägers im Bereich der Rotationsachse. Die Rotationsachse kann sich dabei an einem Ende der kurzen Seite des Trägers befinden, also in einer Ecke der rechteckigen Form. Insbesondere wird eine mechanische Belastung oder Blockade oder Bewegung der elektrischen Anschlussleitung bei der Schwenkbewegung von der ersten Schwenkstellung in die zweite Schwenkstellung durch die räumliche Nähe zur Rotationsachse vermieden oder verringert.

Insbesondere kann das Hardwaremodul eine Hohlachse aufweisen. Dabei kann beispielsweise die Rotationsachse durch die Hohlachse verlaufen, beispielsweise durch ein Zentrum der Hohlachse. Die Hohlachse kann beispielsweise die elektrische Anschlussleitung zumindest teilweise umschließen.

Ein Vorteil dieser Ausführungsformen ist, dass unerwünschte Bewegungen und/oder Belastungen der elektrischen Anschlussleitung durch die Rotationsbewegung des Hardwaremoduls vermieden oder verringert werden können. Dazu kann die elektrische Anschlussleitung in einer räumlichen Nähe zur Rotationsachse verlaufen und/oder durch die Hohlachse verlaufen. Insbesondere muss damit die elektrische Anschlussleitung beispielsweise weder von der Steuerungselektronik noch von dem wenigstens einen Aktorelement gelöst werden, wenn sich das Hardwaremodul von der ersten Schwenkstellung in die zweite Schwenkstellung bewegt.

Gemäß zumindest einer weiteren Ausführungsform weist die Vorrichtung das wenigstens eine Aktorelement auf.

Gemäß zumindest einer weiteren Ausführungsform ist das wenigstens eine Aktorelement dazu eingerichtet, die Patientenauflage gemäß wenigstens einer translatorischen Achse und/oder gemäß wenigstens einer rotatorischen Achse zu bewegen.

Insbesondere kann sich das wenigstens eine Aktorelement innerhalb der Säule befinden und/oder innerhalb beziehungsweise an der Patientenauflage befinden. Beispielsweise kann das wenigstens eine Aktorelement dazu eingerichtet sein, die Patientenauflage gemäß drei translatorischer Achsen und/oder gemäß drei rotatorischer Achsen zu bewegen. Insbesondere kann bei bestimmungsgemäßer Aufstellung die Säule während einer Bewegung der Patientenauflage in ihrer Position verharren.

Ein Vorteil dieser Ausführungsformen ist, dass eine flexible Lageveränderung der Patientenauflage möglich ist und damit der Patient, der sich auf der Patientenauflage befindet, in eine für die jeweilige Untersuchung und/oder Behandlung vorteilhafte Lage versetzt werden kann. Insbesondere kann dabei eine Lage des Patienten relativ zur Patientenauflage unverändert bleiben.

Gemäß zumindest einer weiteren Ausführungsform weist die Säule einen Bodenrahmen auf, der sich an einer Unterseite der Vorrichtung befindet. Außerdem ist das Hardwaremodul um die Rotationsachse schwenkbar an dem Bodenrahmen befestigt.

Insbesondere kann sich die Unterseite der Vorrichtung bei bestimmungsgemäßem Aufbau in Kontakt mit der Aufstellfläche der Vorrichtung, also insbesondere einem Gebäudeboden und/oder einem Boden eines Behandlungsraums oder eines Untersuchungsraums, befinden. Beispielsweise kann dazu der Bodenrahmen mit der Aufstellfläche verschraubt werden. Insbesondere ist das Hardwaremodul lediglich an dem Bodenrahmen befestigt und nicht zusätzlich mit einer weiteren Komponente der Säule befestigt.

Insbesondere kann sich die Patientenauflage an einer Oberseite der Vorrichtung befinden, wobei die Oberseite der Unterseite gegenüberliegt.

Ein Vorteil dieser Ausführungsformen ist, dass sich das Hardwaremodul in einer räumlichen Entfernung von der Patientenauflage befindet. Damit kann beispielsweise eine Beeinflussung der Steuerungselektronik durch zu der Patientenauflage geführte elektromagnetische Strahlung reduziert werden. Beispielsweise kann somit eine ungewollte Beeinträchtigung oder Beschädigung der Steuerungselektronik durch die elektromagnetische Strahlung und/oder ionisierende Strahlung vermieden werden. Ebenso kann eine Funktion eines Bestrahlungssystems oder eines Bildgebungssystems durch die Steuerungselektronik, insbesondere aufgrund durch die Steuerungselektronik erzeugter Hochfrequenzsignale, vermieden werden.

Gemäß zumindest einer weiteren Ausführungsform weist die Vorrichtung eine elektromagnetische Abschirmvorrichtung auf, wobei sich das Hardwaremodul in der ersten Schwenkstellung auf der ersten Seite der elektromagnetischen Abschirmvorrichtung befindet und die Patientenauflage auf einer zweiten Seite der elektromagnetischen Abschirmvorrichtung befindet, wobei die zweite Seite sich gegenüber der ersten Seite befindet.

Beispielsweise kann die Vorrichtung durch Anwendung einer Bestrahlungseinheit oder einer Bildgebungseinheit elektromagnetischer Strahlung ausgesetzt sein, insbesondere wenn sich das Hardwaremodul in der ersten Schwenkstellung befindet. Die elektromagnetische Abschirmvorrichtung kann beispielsweise innerhalb des Innenraums der Säule integriert sein. Ebenso kann die elektromagnetische Abschirmvorrichtung Teil des Bodenrahmens sein.

Ein Vorteil dieser Ausführungsformen ist, dass das Hardwaremodul, insbesondere die Steuerungselektronik, aufgrund der elektromagnetischen Abschirmvorrichtung vor der auftretenden elektromagnetischen Strahlung besser geschützt werden kann. Ebenso kann eine Beeinflussung der Bestrahlungseinheit und/oder der Bildgebungseinheit durch die Steuerungselektronik durch die elektromagnetische Abschirmvorrichtung weiter reduziert werden. Dadurch kann einerseits eine Qualität der Strahlungstherapie und/oder der erzeugten Bilddaten erhöht werden und andererseits eine Lebensdauer der Steuerungselektronik erhöht werden.

Gemäß zumindest einer weiteren Ausführungsform enthält die elektromagnetische Abschirmvorrichtung eine Metallplatte, beispielsweise eine Stahlplatte.

Insbesondere kann bei bestimmungsgemäßem Aufbau die Metallplatte in dem Bodenrahmen angeordnet sein oder mit dem Bodenrahmen verbunden sein.

Ein Vorteil dieser Ausführungsformen ist, dass die Metallplatte eine hohe elektromagnetische Abschirmwirkung aufweisen kann. Gleichzeitig kann sich ein Gewicht der Metallplatte vorteilhaft auf eine Stabilität der Vorrichtung auswirken, insbesondere während einer Lageveränderung der Patientenauflage durch das wenigstens eine Aktorelement.

Gemäß einem weiteren Aspekt der Erfindung wird ein Bestrahlungssystem vorgestellt. Das Bestrahlungssystem weist ein Bestrahlungsgerät zur Erzeugung von ionisierender Strahlung und eine Vorrichtung zur Lagerung eines Patienten gemäß der Erfindung auf.

Gemäß zumindest einer Ausführungsform kann das Bestrahlungsgerät dazu eingerichtet sein eine Gammastrahlung und/oder eine Röntgenstrahlung und/oder eine Neutronenstrahlung und/oder eine Protonenstrahlung und/oder eine schwere Ionenstrahlung und/oder eine Elektronenstrahlung zu erzeugen.

Gemäß einem weiteren Aspekt der Erfindung wird ein Bildgebungssystem vorgestellt. Das Bildgebungssystem weist eine Bildgebungseinheit zur Erzeugung von Bilddaten und eine Vorrichtung zur Lagerung eines Patienten gemäß der Erfindung auf.

Gemäß zumindest einer Ausführungsform entspricht die Bildgebungseinheit einer röntgenbasierten Bildgebungseinheit und/oder einer Positronenemissions-Tomographie-Bildgebungseinheit (PET-Bildgebungseinheit) und/oder einer Magnetresonanztomographie-Bildgebungseinheit (MRT-Bildgebungseinheit).

Insbesondere kann die röntgenbasierte Bildgebungseinheit einer Computertomographie-Bildgebungseinheit (CT-Bildgebungseinheit) und/oder einem C-Bogen-Gerät und/oder einem klassischen Röntgengerät entsprechen.

Weitere Ausführungsformen des erfindungsgemäßen Bildgebungssystems folgen unmittelbar aus den verschiedenen Ausgestaltungen der erfindungsgemäßen Vorrichtung. Insbesondere lassen sich einzelne Merkmale und entsprechende Erläuterungen sowie Vorteile bezüglich der verschiedenen Ausführungsformen zu der erfindungsgemäßen Vorrichtung analog auf entsprechende Ausführungsformen des erfindungsgemäßen Bildgebungssystems übertragen.

Weitere Merkmale und Merkmalskombinationen der Erfindung ergeben sich aus den Figuren und deren Beschreibung sowie aus den Ansprüchen. Insbesondere müssen weitere Ausführungsformen der Erfindung nicht unbedingt alle Merkmale eines der Ansprüche enthalten. Weitere Ausführungsformen der Erfindungen können Merkmale oder Merkmalskombinationen aufweisen, die nicht in den Ansprüchen genannt sind.

Die Erfindung wird im Folgenden anhand konkreter Ausführungsbeispiele und zugehöriger schematischer Zeichnungen näher erläutert. In den Figuren können gleiche oder funktionsgleiche Elemente mit denselben Bezugszeichen versehen sein. Die Beschreibung gleicher oder funktionsgleicher Elemente wird gegebenenfalls nicht notwendigerweise bezüglich verschiedener Figuren wiederholt.

Dabei zeigen:
- FIG 1: eine schematische Darstellung einer beispielhaften Ausführungsform eines erfindungsgemäßen Bestrahlungssystems oder eines erfindungsgemäßen Bildgebungssystems; und
- FIG 2: eine schematische Darstellung einer beispielhaften Ausführungsform einer erfindungsgemäßen Vorrichtung zur Lagerung eines Patienten; und
- FIG 3: eine schematische Darstellung eines Teils einer weiteren beispielhaften Ausführungsform einer erfindungsgemäßen Vorrichtung zur Lagerung eines Patienten; und
- FIG 4: eine schematische Darstellung von Details der Ausführungsform aus FIG 2; und
- FIG 5: eine schematische Darstellung eines Teils einer weiteren beispielhaften Ausführungsform einer erfindungsgemäßen Vorrichtung zur Lagerung eines Patienten; und
- FIG 6: eine schematische Darstellung eines Teils einer weiteren beispielhaften Ausführungsform einer erfindungsgemäßen Vorrichtung zur Lagerung eines Patienten; und
- FIG 7: eine schematische Darstellung einer weiteren beispielhaften Ausführungsform einer erfindungsgemäßen Vorrichtung zur Lagerung eines Patienten.

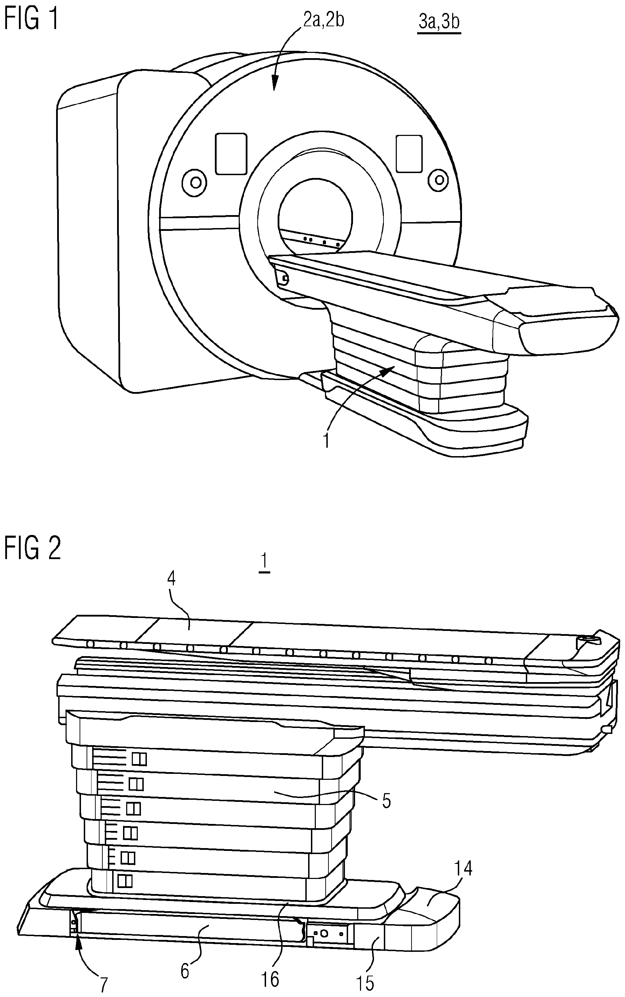

In FIG 1 wird eine schematische Darstellung einer beispielhaften Ausführungsform eines erfindungsgemäßen Bestrahlungssystems 3a oder eines erfindungsgemäßen Bildgebungssystems 3b gezeigt. Dabei enthält das Bestrahlungssystem 3a ein Bestrahlungsgerät 2a oder das Bildgebungssystem 3b eine Bildgebungseinheit 2b. Ebenso enthält sowohl das Bestrahlungssystem 3a als auch das Bildgebungssystem 3b eine Vorrichtung 1 zur Lagerung eines Patienten. Insbesondere kann die Vorrichtung 1 zur Lagerung des Patienten bei bestimmungsgemäßer Aufstellung unmittelbar vor dem Bestrahlungsgerät 2a oder der Bildgebungseinheit 2b angeordnet sein. Insbesondere kann sich zur Bestrahlung des Patienten und/oder zur Erzeugung von Bilddaten ein Teil der Vorrichtung 1, beispielsweise eine Patientenauflage 4 (siehe FIG 2), innerhalb eines Innenraums der Bildgebungseinheit 2b befinden.

Das Bestrahlungsgerät 2a kann beispielsweise dazu eingerichtet sein, eine Gammastrahlung, eine Röntgenstrahlung, eine Neutronenstrahlung, eine Protonenstrahlung, eine schwere Ionenstrahlung und/oder eine Elektronenstrahlung zu erzeugen
Die Bildgebungseinheit 2b kann beispielsweise eine CT Bildgebungseinheit, eine MRT Bildgebungseinheit, eine PET Bildgebungseinheit, eine C-Arm Bildgebungseinheit oder eine Fluoroskopie Bildgebungseinheit enthalten oder dieser entsprechen.

Insbesondere kann das Bestrahlungssystem 3a zusätzlich zu dem Bestrahlungsgerät 2a ebenso eine Bildgebungseinheit 2b enthalten und damit sowohl dazu eingerichtet sein mittels des Bestrahlungsgeräts 2a Strahlung für eine Strahlentherapie zu erzeugen als auch mittels der Bildgebungseinheit 2b dazu eingerichtet sein, die Bilddaten zu erzeugen.

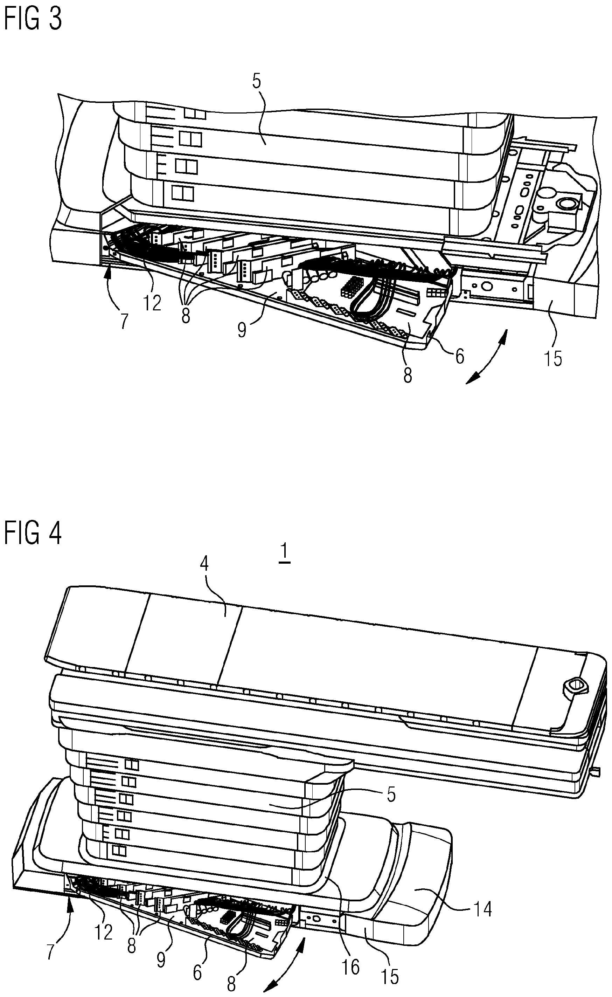

In FIG 2 wird eine schematische Darstellung einer beispielhaften Ausführungsform einer erfindungsgemäßen Vorrichtung 1 zur Lagerung eines Patienten gezeigt. Die Vorrichtung 1 weist eine Säule 5, die Patientenauflage 4 und ein Hardwaremodule 6 auf. Das Hardwaremodul 6 weist dabei eine Steuerungselektronik 8 (siehe FIG 3) zur Ansteuerung wenigstens eines Aktorelements (nicht dargestellt) zur räumlichen Lageveränderung der Patientenauflage 4 auf. Das Hardwaremodul 6 ist um eine Rotationsachse 7 schwenkbar an der Säule 5 befestigt.

Dabei ist das Hardwaremodul 6 in eine erste Schwenkstellung positionierbar, in der sich die Steuerungselektronik 8 in einem Innenraum der Säule 5 befindet. Ebenso ist das Hardwaremodul 6 in eine zweite Schwenkstellung positionierbar, in der die Steuerungselektronik 8 von außerhalb der Säule zugänglich ist. Die Darstellung in FIG 2 entspricht der ersten Schwenkstellung des Hardwaremoduls 6. Mit anderen Worten implementiert die Erfindung eine zweistufige Lösung in Bezug auf die erste und zweite Schwenkstellung des Hardwaremoduls 6.

Die Säule 5 kann in einer weiteren Ausführungsform einen Bodenrahmen 14 aufweisen, der sich an der Unterseite der Vorrichtung 1 befindet. Insbesondere kann der Bodenrahmen 14 bei bestimmungsgemäßer Aufstellung mit einer Aufstellfläche verbunden sein, beispielsweise durch eine Verschraubung oder eine Arretierung, um die Vorrichtung 1 zu stabilisieren. Insbesondere kann bei bestimmungsgemäßer Aufstellung die Säule 5 stationär bezüglich der Aufstellfläche sein und lediglich die Patientenauflage 4 in ihrer Lage veränderlich sein.

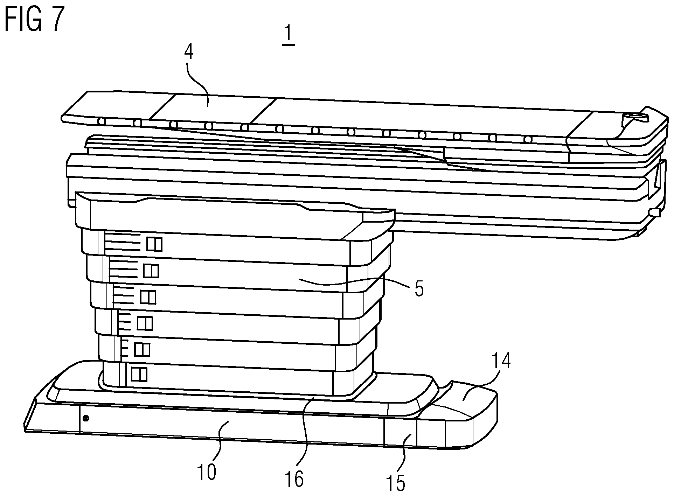

Insbesondere kann die Vorrichtung 1 eine Abdeckung 10 aufweisen (siehe FIG 7), die an der Säule 5 montierbar ist, wenn sich das Hardwaremodul 6 in der ersten Schwenkstellung befindet. Insbesondere kann das Hardwaremodul 6 nur bei demontierter Abdeckung 10 von der ersten Schwenkstellung in die zweite Schwenkstellung schwenkbar sein. FIG 2 zeigt die Vorrichtung 1 beispielhaft bei demontierter Abdeckung 10.

Die Vorrichtung 1 kann darüber hinaus eine weitere Abdeckung 15 enthalten, die mit der Säule 5 verbunden ist und die mit der Abdeckung 10 fluchtet, wenn sich das Hardwaremodul 6 in der ersten Schwenkstellung befindet. Diese Konstellation ist insbesondere in FIG 7 gezeigt.

Die Vorrichtung 1 kann eine elektromagnetische Abschirmvorrichtung 16 aufweisen, wobei sich das Hardwaremodul 6 in der ersten Schwenkstellung auf einer ersten Seite der elektromagnetischen Abschirmvorrichtung 16 befindet und die Patientenauflage 4 auf einer zweiten Seite der elektromagnetischen Abschirmvorrichtung 16, wobei die zweite Seite sich gegenüber der ersten Seite befindet.

Die elektromagnetische Abschirmvorrichtung 16 kann sich beispielsweise auf einer Oberseite des Bodenrahmens 14 befinden, wobei das Hardwaremodul 6 beispielsweise in einem Innenraum des Bodenrahmens 14 angeordnet ist. Die elektromagnetische Abschirmvorrichtung 16 kann beispielsweise die Steuerungselektronik 8 vor Umwelteinflüssen, insbesondere durch elektromagnetische Strahlung, beispielsweise hervorgerufen durch das Bestrahlungsgerät 2a oder die Bildgebungseinheit 2b, schützen. Andererseits kann die elektromagnetische Abschirmvorrichtung 16 auch während der Erzeugung von Bilddaten ein Entstehen von Artefakten oder einer Verminderung eines Signal-Rauschverhältnisses verhindern, indem sie ein abgestrahltes elektromagnetisches Feld der Steuerungselektronik 8 von einer Empfangseinheit der Bildgebungseinheit 2b abschirmt. Insbesondere dazu kann es vorteilhaft sein, dass sich die elektromagnetische Abschirmvorrichtung 16 in unmittelbarer Nähe beispielsweise oberhalb der Steuerungselektronik 8 befindet.

Die elektromagnetische Abschirmvorrichtung 16 kann eine Metallplatte enthalten, beispielsweise eine Stahlplatte, oder der Metallplatte entsprechen. Insbesondere kann die Metallplatte vorteilhaft zur Stabilität der Vorrichtung 1 beitragen, wenn die Metallplatte sich in einem Bereich nahe der Aufstellfläche befindet und damit einen Schwerpunkt der Vorrichtung 1 in einen unteren Bereich der Vorrichtung 1 hervorruft.

FIG 3 zeigt eine schematische Darstellung eines Teils einer weiteren beispielhaften Ausführungsform der erfindungsgemäßen Vorrichtung 1. Dabei ist durch einen Pfeil entlang des Hardwaremoduls 6 eine Schwenkbewegung angedeutet. Insbesondere kann die Schwenkbewegung durch ein Schwenken von der ersten Schwenkstellung in die zweite Schwenkstellung oder umgekehrt stattfinden.

Die Steuerungselektronik 8 kann wenigstens eine elektronische Schaltung aufweisen. In FIG 3 ist dazu eine Vielzahl von elektronischen Schaltungen auf wenigstens einer Leiterplatte dargestellt. Die Darstellung in FIG 3 kann beispielsweise der zweiten Schwenkstellung entsprechen oder einer Zwischenstellung zwischen der ersten Schwenkstellung und der zweiten Schwenkstellung.

Insbesondere kann das Hardwaremodul 6 eine elektrische Anschlussleitung 12 zum Anschluss der Steuerungselektronik 8 an das wenigstens eine Aktorelement aufweisen. Die elektrische Anschlussleitung 12 verläuft von der Rotationsachse entlang einer Oberfläche des Trägers 9 zu der Steuerungselektronik 8. Insbesondere verläuft die elektrische Anschlussleitung 12 in einem Bereich der Rotationsachse 7. Zumindest kann ein Teil der elektrischen Anschlussleitung 12, der sich an einem Übergang von dem Innenraum der Säule 5 zu dem Hardwaremodul 6 befindet in unmittelbarer Nähe der Rotationsachse 7 verlaufen. Die elektrische Anschlussleitung 12 kann dabei weiter in den Innenraum der Säule 5 verlaufen und dort zu jedem des wenigstens einen Aktorelements geführt sein.

FIG 4 zeigt eine schematische Darstellung der beispielhaften Ausführungsform aus FIG 2. Dabei ist in dieser Darstellung das Hardwaremodul in der zweiten Schwenkstellung dargestellt. Soweit nicht anderweitig beschrieben gelten für diese Ausführungsform ein Teil der Erläuterungen oder alle Erläuterungen aus FIG 2 und FIG 3. Dabei ist durch einen Pfeil entlang des Hardwaremoduls 6 die Schwenkbewegung angedeutet.

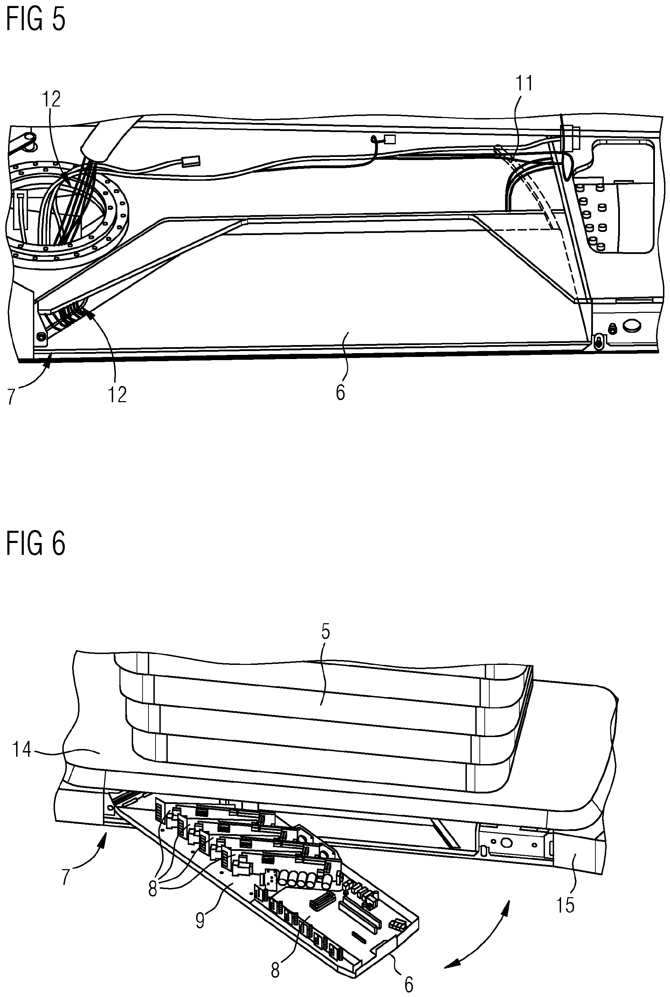

FIG 5 zeigt eine schematische Darstellung eines Teils einer weiteren beispielhaften Ausführungsform einer erfindungsgemäßen Vorrichtung 1. Das Hardwaremodul 6 befindet sich in dieser Darstellung in der ersten Schwenkstellung.

Das Hardwaremodul 6 kann ein Anschlagelement 11 aufweisen, das mit dem Träger 9 verbunden ist und das die Schwenkbewegung des Hardwaremoduls 6 von der ersten Schwenkstellung in die zweite Schwenkstellung in der zweiten Schwenkstellung begrenzt.

Das Anschlagelement 11 kann beispielsweise in einer Führungsschiene oder in einer Arretierung verlaufen, die in dem Innenraum der Säule 5 angeordnet sind. Insbesondere in der ersten Schwenkstellung kann das Anschlagelement 11 von einem Außenraum der Säule 5 nicht zugänglich sein. Insbesondere kann das Anschlagelement 11 zerstörungsfrei von dem Träger 9 demontierbar sein und/oder aus der Führungsschiene oder aus der Arretierung lösbar sein.

Insbesondere zeigt die Darstellung in FIG 5 eine Führung der elektrischen Anschlussleitung 12 von dem Innenraum der Säule 5 durch eine kreisförmige Durchführung, die ebenso in dem Innenraum der Säule 5 angeordnet ist, zu dem Hardwaremodul 6, insbesondere auf den Träger 9.

FIG 6 zeigt eine schematische Darstellung eines Teils einer weiteren beispielhaften Ausführungsform einer erfindungsgemäßen Vorrichtung 1. Im Vergleich zu FIG 5 ist das Anschlagelement 11 in der Darstellung in FIG 6 nicht dargestellt, da es beispielsweise demontiert ist. Das Hardwaremodul 6 kann beispielsweise nur bei demontiertem Anschlagelement 11 in eine dritte Schwenkstellung positionierbar sein, in der die Steuerungselektronik 8 von dem Träger 9 zerstörungsfrei demontierbar ist. Das Hardwaremodul 6 befindet sich in der Darstellung in FIG 6 in der dritten Schwenkstellung. Mit anderen Worten kann damit eine dreistufige Lösung in Bezug auf die erste, zweite und dritte Schwenkstellung des Hardwaremoduls 6 implementiert werden. Dabei ist durch einen Pfeil entlang des Hardwaremoduls 6 die Schwenkbewegung angedeutet.

Beispielsweise kann das Hardwaremodul 6 von der ersten Schwenkstellung über die zweite Schwenkstellung hinaus in die dritte Schwenkstellung geschwenkt werden, wenn das Anschlagelement 11 demontiert ist. Ebenso kann das Hardwaremodul 6 von der dritten Schwenkstellung über die zweite Schwenkstellung hinaus in die erste Schwenkstellung geschwenkt werden. Zusätzlich kann das Hardwaremodul 6 in manchen Ausführungsformen weiter über die erste Schwenkstellung hinaus in den Innenraum der Säule 5 geschwenkt werden. In der zweiten Schwenkstellung kann beispielsweise das Anschlagelement 11 montiert werden, sodass das Hardwaremodul 6 damit in der Schwenkbewegung begrenzt werden kann.

FIG 7 zeigt eine schematische Darstellung einer weiteren beispielhaften Ausführungsform der erfindungsgemäßen Vorrichtung 1. Dabei ist in dieser Darstellung das Hardwaremodul 6 in der ersten Schwenkstellung dargestellt. Soweit nicht anderweitig beschrieben, gelten für diese Ausführungsform ein Teil der Erläuterungen oder alle Erläuterungen aus den übrigen Darstellungen.

Insbesondere zeigt die Darstellung die Abdeckung 10, die an der Säule 5 montiert ist und mit der weiteren Abdeckung 15 fluchtet. Ebenso ist eine mögliche Anordnung der elektromagnetischen Abschirmvorrichtung 16 gezeigt, die sich oberhalb des Hardwaremoduls 6 an dem Bodenrahmen 14 befindet.

Unabhängig vom grammatikalischen Geschlecht eines bestimmten Begriffes sind Personen mit männlicher, weiblicher oder anderer Geschlechteridentität mit umfasst.

## Patentansprüche

1. Vorrichtung (1) zur Lagerung eines Patienten, wobei
- die Vorrichtung (1) eine Säule (5), eine Patientenauflage (4) und ein Hardwaremodul (6) aufweist;
- das Hardwaremodul (6) eine Steuerungselektronik (8) zur Ansteuerung wenigstens eines Aktorelements zur räumlichen Lageveränderung der Patientenauflage (4) aufweist;
- das Hardwaremodul (6) einen Träger (9) aufweist, auf dem die Steuerungselektronik (8) angeordnet ist;
- das Hardwaremodul (6) um eine Rotationsachse (7) schwenkbar an der Säule (5) befestigt ist;
- das Hardwaremodul (6) in eine erste Schwenkstellung positionierbar ist, in der sich die Steuerungselektronik (8) in einem Innenraum der Säule (5) befindet; und
- das Hardwaremodul (6) in eine zweite Schwenkstellung positionierbar ist, in der die Steuerungselektronik (8) von außerhalb der Säule (5) zugänglich ist.

2. Vorrichtung (1) nach Anspruch 1, wobei das Hardwaremodul (6) ein Anschlagelement (11) aufweist, das mit dem Träger (9) verbunden ist und das eine Schwenkbewegung des Hardwaremoduls (6) von der ersten Schwenkstellung in die zweite Schwenkstellung in der zweiten Schwenkstellung begrenzt.

3. Vorrichtung (1) nach Anspruch 2, wobei
- das Anschlagelement (11) zerstörungsfrei von dem Träger (9) demontierbar ist und das Hardwaremodul (6) nur bei demontiertem Anschlagelement (11) in eine dritte Schwenkstellung positionierbar ist, in der die Steuerungselektronik (8) von dem Träger (9) zerstörungsfrei demontierbar ist; oder
- das Anschlagelement (11) aus einer Arretierung an der Säule lösbar ist und das Hardwaremodul (6) nur bei aus der Arretierung gelöstem Anschlagelement (11) in eine dritte Schwenkstellung positionierbar ist, in der die Steuerungselektronik (8) von dem Träger (9) zerstörungsfrei demontierbar ist.

4. Vorrichtung (1) nach Anspruch 3, wobei
- das Hardwaremodul (6) nur bei demontiertem Anschlagelement (11) von der ersten Schwenkstellung über die zweite Schwenkstellung hinaus in die dritte Schwenkstellung schwenkbar ist; oder
- das Hardwaremodul (6) nur bei aus der Arretierung gelöstem Anschlagelement (11) von der ersten Schwenkstellung über die zweite Schwenkstellung hinaus in die dritte Schwenkstellung schwenkbar ist.

5. Vorrichtung (1) nach einem der vorherigen Ansprüche, wobei
- die Vorrichtung (1) eine Abdeckung (10) aufweist, die an der Säule (5) montierbar ist, wenn sich das Hardwaremodul (6) in der ersten Schwenkstellung befindet; und
- das Hardwaremodul (6) nur bei demontierter Abdeckung (10) von der ersten Schwenkstellung in die zweite Schwenkstellung schwenkbar ist.

6. Vorrichtung (1) nach einem der Ansprüche 1 bis 4, wobei die Vorrichtung (1) eine Abdeckung (10) aufweist, die mit dem Hardwaremodul (6) verbunden ist und die zusammen mit dem Hardwaremodul (6) von der ersten Schwenkstellung in die zweite Schwenkstellung schwenkbar ist.

7. Vorrichtung (1) nach Anspruch 5 oder 6, wobei die Vorrichtung (1) eine weitere Abdeckung (15) aufweist, die mit der Säule (5) verbunden ist und die mit der Abdeckung (10) fluchtet, wenn sich das Hardwaremodul (6) in der ersten Schwenkstellung befindet.

8. Vorrichtung (1) nach einem der vorherigen Ansprüche, wobei die Rotationsachse (7) entlang einer Vertikalrichtung der Vorrichtung (1) verläuft.

9. Vorrichtung (1) nach einem der vorherigen Ansprüche, wobei
- das Hardwaremodul (6) eine elektrische Anschlussleitung (12) zum Anschluss der Steuerungselektronik (8) an das wenigstens eine Aktorelement aufweist; und
- die elektrische Anschlussleitung (12) von der Rotationsachse (7) entlang einer Oberfläche des Trägers (9) zu der Steuerungselektronik (8) verläuft.

10. Vorrichtung (1) nach einem der vorherigen Ansprüche, wobei
- die Vorrichtung (1) das wenigstens eine Aktorelement aufweist; und
- das wenigstens eine Aktorelement dazu eingerichtet ist, die Patientenauflage (4) gemäß wenigstens einer translatorischen Achse und/oder gemäß wenigstens einer rotatorischen Achse zu bewegen.

11. Vorrichtung (1) nach einem der vorherigen Ansprüche, wobei
- die Säule (5) einen Bodenrahmen (14) aufweist, der sich an einer Unterseite der Vorrichtung (1) befindet; und
- das Hardwaremodul (6) um die Rotationsachse (7) schwenkbar an dem Bodenrahmen (14) befestigt ist.

12. Vorrichtung (1) nach Anspruch 11, wobei die Vorrichtung (1) eine elektromagnetische Abschirmvorrichtung (16) aufweist, wobei sich das Hardwaremodul (6) in der ersten Schwenkstellung auf einer ersten Seite der elektromagnetischen Abschirmvorrichtung (16) befindet und sich die Patientenauflage (4) auf einer der ersten Seite gegenüberliegenden zweiten Seite der elektromagnetischen Abschirmvorrichtung (16) befindet.

13. Vorrichtung (1) nach Anspruch 12, wobei die elektromagnetische Abschirmvorrichtung (16) eine Metallplatte enthält.

14. Bestrahlungssystem (3a), aufweisend ein Bestrahlungsgerät (2a) zur Erzeugung von ionisierender Strahlung und eine Vorrichtung (1) zur Lagerung eines Patienten nach einem der Ansprüche 1 bis 13.

15. Bildgebungssystem (3b), aufweisend eine Bildgebungseinheit (2b) zur Erzeugung von Bilddaten und eine Vorrichtung (1) zur Lagerung eines Patienten nach einem der Ansprüche 1 bis 13.
